# EUROPÄISCHE PATENTSCHRIFT

(11) **EP 1 796 750 B1**
(45) Veröffentlichungstag und Bekanntmachung des Hinweises auf die Patenterteilung: **08.01.2014**
(21) Anmeldenummer: 05794777.2
(22) Anmeldetag: 22.09.2005
(51) Int. Cl.: A61L 27/18, A61L 27/44, A61L 27/56, A61L 27/54

(54) **ANTIMIKROBIELLES IMPLANTAT MIT EINER FLEXIBLEN POROESEN STRUKTUR, AUSGEBILDET ALS SPRUEHVLIES**
ANTIMICROBIAL IMPLANT WITH A FLEXIBLE POROUS STRUCTURE IN THE FORM OF A SPRAYED NON-WOVEN
IMPLANT ANTIMICROBIEN COMPRENANT UNE STRUCTURE POREUSE SOUPLE DANS UN FORME D'UN TISSU NON TISSÉ

(30) Priorität: 24.09.2004 DE 102004047568
(43) Veröffentlichungstag der Anmeldung: 20.06.2007
(73) Patentinhaber: Aesculap AG, 78532 Tuttlingen (DE)
(72) Erfinder: GOLDMANN, Helmut, 34119 Kassel (DE); LANGANKE, Dennis, 72074 Tübingen (DE); FRIEDRICH, Volker, 78579 Neuhausen (DE)
(74) Vertreter: Patentanwälte Ruff, Wilhelm, Beier, Dauster & Partner
(86) Internationale Anmeldenummer: PCT/EP2005/010256
(87) Internationale Veröffentlichungsnummer: WO 2006/032497

(56) Entgegenhaltungen:
- WO-A-96/03165
- WO-A-98/57680
- WO-A-03/045448
- US-A- 5 019 096
- US-A1- 2001 010 022
- PATENT ABSTRACTS OF JAPAN Bd. 2003, Nr. 12, 5. Dezember 2003 (2003-12-05) & JP 2004 256794 A (TAKASHIMA:KK), 16. September 2004 (2004-09-16)
- PATENT ABSTRACTS OF JAPAN Bd. 2002, Nr. 04, 4. August 2002 (2002-08-04) & JP 2001 340375 A (TOYOBO CO LTD), 11. Dezember 2001 (2001-12-11)
- PATENT ABSTRACTS OF JAPAN Bd. 1995, Nr. 01, 28. Februar 1995 (1995-02-28) & JP 06 293611 A (ASAHI CHEM IND CO LTD), 21. Oktober 1994 (1994-10-21) -& DATABASE WPI Section Ch, Week 199502 Derwent Publications Ltd., London, GB; Class A60, AN 1995-009520 XP002367481 & JP 06 293611 A (ASAHI KASEI KOGYO KK) 21. Oktober 1994 (1994-10-21)

## Beschreibung

Die Erfindung betrifft ein antimikrobielles Implantat mit einer flexiblen porösen Struktur aus einem bioverträg lichen Kunststoff.

In jüngerer Zeit ist man vermehrt dazu übergegangen, Implantate, wie Gefäßprothesen, antimikrobiell auszurüsten, um Primärinfektionen oder auch Sekundärinfektionen zu unterdrücken. So ist in der US 5,019,096 ein Verfahren beschrieben, um Implantate unterschiedlichster Art infektionsresistent zu machen. Hierzu werden die Implantate oder sonstige medizinische Artikel, wie Katheter und Gummihandschuhe, auf ihrer Oberfläche mit einem Beschichtungsmaterial versehen, welches synergistische Mengen eines Silbersalzes und eines Biguanids enthält.

Aus der EP 0 184 465 ist ein thermoplastisches Polyurethanprodukt bekannt, bei dem auf einem Substrat mindestens eine Schicht aus eine m thermoplastischen Polyurethan vorgesehen ist, in der antithrombogene Materialien und antibiotische Materialien enthalten sein können, um nach einer Implantation gegebenenfalls Thrombosen und Infektionen zu vermeiden.

Die DE 100 43 151 A1 beschreibt einen Knochenzement mit antimikrobieller Wirksamkeit. Zur Erzeugung dieser Wirksamkeit ist nano- bis mikrodisperses Silber in Mengen bis zu 2 Gew:% im Zementmaterial vermischt. In der DE 43 44 306 A1 ist ein Kunststoffgegenstand beschrieben, der auf und/oder unter der Oberfläche metallisches Silber aufweist. Der Gegenstand, z.B. ein Schlauch, kann einen Überzug aus metallischem Silber aufweisen. Es ist auch möglich, Silberpulver in zu extrudierende Massen einzuarbeiten. Aus der WO 2004/060210 A1 ist es zur Herstellung von radioopaken Gefäßprothesen bekannt, PTFE- Partikel vor ihrer Verarbeitung zu Prothesen aus expandiertem PTFE mit Partikeln aus radioopakem Material, z.B. Silber, zu vermischen. Abgesehen davon, dass hier die Gefahr der Abgabe von Silberpartikeln in den Blutstrom besteht, da die Silberpartikel nicht in die PTFE-Partikel selbst eingelagert sind, sind die Variationsmöglichkeiten beschränkt.

Die WO 96/03165 A1 beschreibt Hernienimplantate mit einer porösen Struktur in Form einer mikroporösen Membran oder einer Maschenware. Die poröse Struktur kann mit einem resorbierbaren Material imprägniert werden, das ein antimikrobielles Mittel enthält.

Bei der EP 0 633 032 B1 wird eine Gefäßprothese aus einem schlauchförmigen porösen Körper dadurch antibakteriell ausgerüstet, dass ein Schlauch, eine Faser oder eine Folie aus einem polymeren Material, das mit einer antibakteriellen Substanz versehen ist, derart um die Gefäßprothese gewickelt wird, dass die Oberfläche der Gefäßprothese teilweise unbedeckt bleibt.

In der WO 98/31404 werden Prothesen aus biologischem Gewebe oder biologischem Polymer dadurch antimikrobiell ausgerüstet, dass die Prothese mit exogenem Material versehen wird, das Silberionen enthält.

Aus der US 5,019,096 geht ein Verfahren zur Herstellung von antimikrobiellen Materialien hervor, bei welchem ein antimikrobielles Mittel, enthaltend ein Silbersalz und ein Biguanid, in eine polymere Matrix eingebracht werden.

In der WO 98/57680 A2 werden medizinische Produkte auf Basis einen porösen Materials sowie eines wasserunlöslichen, therapeutischen Silbersalzes beschrieben.

Gegenstand der US 2001/0010022 A1 ist ein medizinisches Produkt mit einer Melt-Blown-Faserstruktur, wobei in das Produkt antimikrobielle Wirkstoffe eingebracht werden können.

Gegenstand der JP 2004 256794 A ist ein Polyurethanschaum, der anorganische antibakterielle Wirkstoffe wie beispielsweise Silber und Titandioxid aufweist.

Die WO 96/03165 A1 betrifft Prothesen für die Abdominalwand, welche ein antimikrobielles Mittel aufnehmen bzw. abgeben können, wobei es sich bei den Prothesenmaterialien unter anderem um non-woven Materialien handeln kann.

Aus der JP 2001 340375 A ist ein mehrschichtiges Wundmaterial bekannt, wobei das Wundmaterial eine Schicht aus einem non-woven Material sowie ein antibakterielles Mittel aufweist.

Die JP 06 293611 A betrifft ein antimikrobielles Material auf Basis eines polymeren Grundmaterials sowie von ultrafeinen metallischen Partikeln, die in dem Grundmaterial dispergiert vorliegen.

In der WO 03/045448 A1 werden Polymersubstrate offenbart, welche mit einer antimikrobiellen Substanz, bei welcher es sich unter anderem um Metalle wie Silber, Zink, Kupfer oder Mischungen davon handeln kann, imprägniert werden.

Der Erfindung liegt die Aufgabe zugrunde, ein antimikrobielles Implantat mit einer flexiblen porösen Struktur aus einem bioverträglichen Kunststoff in einfacher Weise derart herzustellen, dass eine über lange Zeit andauernde antimikrobielle Wirkung erzielt wird. Dies wird dadurch erreicht, dass der antimikrobielle Wirkstoff im Kunststoff des Implantats vorliegt.

Gegenstand der Erfindung ist somit ein antimikrobielles Implantat mit einer flexiblen porösen Vliesstruktur aus einem bioverträglichen Kunststoff, in dem Partikel aus mindestens einem antimikrobiellen Wirkstoff enthalten sind, wobei das Implantat als Sprühvlies ausgebildet ist. Dadurch, dass der antimikrobielle Wirkstoff im Kunststoff enthalten ist, d.h. in den Fasern des Faservlieses, ist die Herstellung des porösen Implantats mit bekannten Techniken möglich, ohne dass nachträglich besondere Beschichtungen angebracht werden müssen. Ein wesentlicher Vorteil der Erfindung liegt auch darin, dass die Poren des erfindungsgemäßen Implantats frei liegen, d.h. nicht durch irgendwelche Beschichtungsmaterialien, die den antimikrobiellen Wirkstoff enthalten oder durch die Wirkstoffpartikel selbst, zugesetzt sind. Das erfindungsgemäße Implantat ist auch in üblicher Weise handhabbar bzw. weiterverarbeitbar. So kann es nach herkömmlichen Sterilisationsverfahren sterilisiert werden, insbesondere mit Ethylenoxid.

Das erfindungsgemäße Implantat kann als flächiges Implantat ausgebildet sein, beispielsweise als Patch, insbesondere zum Abdecken oder Verschließen von Wunden. So kann es als Hernienimplantat ausgebildet sein. Mit Vorteil ist das Implantat auch als Hohlorgan zum Ersatz von Hohlorganen des menschlichen oder tierischen Körpers ausgebildet. So ist das erfindungsgemäße Implantat bei einer bevorzugten Ausführungsform der Erfindung als Gefäßprothese ausgebildet. Geeignete Hohlorgane nach der Erfindung sind als flexible poröse Schläuche ausgebildet, insbesondere mit einem Innendurchmesser von 2 bis 38 mm. Als besonders geeignet erweisen sich kleinlumige Schläuche mit einem Innendurchmesser von 2 bis 24 mm, insbesondere von 2 bis weniger als 14 mm. Die poröse Struktur ist vorzugsweise eine mikroporöse Struktur, insbesondere eine solche mit einer Luftdurchlässigkeit von 5 bis 500 ml/cm²/Minute bei einer Druckdifferenz von 1,2 kPa. Durch geeignete Herstellungsverfahren lässt sich die Porosität der insbesondere dreidimensionalen Struktur einstellen. So können bevorzugte Bereiche der Porosität im Bereich von 10 bis 200 ml, insbesondere 10 bis 100 ml oder 100 bis 200 ml/cm²/Minute eingestellt werden.

Der bioverträgliche Kunststoff ist mit Vorteil ein nicht resorbierbarer Kunststoff. Dadurch ist das erfindungsgemäße Implantat langlebig. Es hat sich gezeigt, dass der Kunststoff zur Entfaltung der antimikrobiellen Wirksamkeit ausreichende Mengen an antimikrobiellem Wirkstoff freigeben kann, auch wenn er nicht abbaubar ist. Als bioverträglicher Kunststoff eignen sich mit Vorteil thermisch plastifizierbare Kunststoffe, insbesondere Thermoplaste. Auch in organischen Lösungsmitteln lösbare Kunststoffe sind geeignet. So kommen als Kunststoffe mit Vorteil Polyester, z.B. Dacron^{®}, Polypropylen, Polyvinylidenfluorid, Polyamid, Polyetherketone, Polytetrafluorethylen und Polyurethan in Frage. Für bestimmte Anwendungen sind Kunststoffe mit hydrophoben Eigenschaften bevorzugt, wie Polyurethan und Polypropylen.

Das erfindungsgemäße Implantat ist vorzugsweise dünnwandig ausgebildet und besitzt insbesondere eine Wandstärke von 0,1 bis 2 mm, vorzugsweise 0,3 bis 0,6 mm.

Gemäß der Erfindung wird die flexible poröse Vlies-Struktur von einem Wandungsmaterial gebildet, das ein Non-woven ist, d.h. weder eine Maschenware, noch ein Gewebe. Bei dem durch Materialauftrag hergestellten Faservlies handelt es sich um ein Sprühvlies. Die Verfahren zur Herstellung solcher Vliese sind bekannt.

Die Partikeln aus dem mindestens einen antimikrobiellen Wirkstoff können mit Vorteil im wesentlichen homogen im Kunststoff verteilt sein. Die Homogenität bezieht sich vorzugsweise auf den Kunststoff jeder Einzelfaser der Vliesstruktur. Je nach Lage der Fasern könen diese eine andere Wirkstoffkonzentration aufweisen als Fasern an einer anderen Lage der Vliesstruktur. Es kann bei besonderen Ausführungsformen erwünscht sein, dass die Partikel aus dem mindestens einen antimikrobiellen Wirkstoff an mindestens einer Oberfläche des Implantats angereichert sind. Derartige Ausgestaltungen sind mit der Sprühtechnik in einfacher Weise zu gestalten. Es ist möglich, bei der Herstellung der Vliesstruktur mit verschiedenen Kunststofflösungen oder -schmelzen zu arbeiten, die sich insbesondere durch die Konzentration an den Partikeln aus dem antimikrobiellen Wirkstoff unterscheiden. Die Lösungen oder Schmelzen können aus verschiedenen Sprühpistolen ausgetragen werden. Dadurch ist ein schichtweiser Aufbau der Implantate möglich. Die unterschiedlichen Konzentrationen können durch verschiedene Mengen an Partikeln und/oder durch verschiedene Größen der Partikel in Kunststoff verwirklicht sein.

Die Größe der Partikel mit antimikrobieller Wirkung liegt vorzugsweise im Nanobereich. Es kommen Partikelgrößen von 50 bis 100 nm besonders in Frage. Die Partikel können zu Agglomeraten verbunden sein, die insbesondere eine Größe von 5 bis 10 µm haben können. Als Material für die Partikel mit antimikrobieller Wirkung kommen insbesondere Metalle oder Metalllegierungen mit antimikrobieller Wirkung in Frage, wobei metallisches Silber besonders bevorzugt ist. Die hervorragende antimikrobielle Wirkung von Silber ist bekannt. Geeignete Silberpartikel sind beispielsweise in der WO 02/17984 A1 beschrieben. Der Gehalt an den Partikeln aus dem mindestens einen antimikrobiellen Wirkstoff im Kunststoff des erfindungsgemäßen Implantats kann in weiten Grenzen variieren. Bevorzugt liegt der Gehalt bei 0,1 bis 10 Gew.%, insbesondere 0,2 bis 5 Gew.%. In der Regel sind Mengen von 0,5 bis 2,5 Gew.% besonders geeignet.

Die Erfindung betrifft auch ein Verfahren zur Herstellung des erfindungsgemäßen Implantates. Nach dem Verfahren wird der Kunststoff mit den Partikeln aus dem antimikrobiellen Wirkstoff spätestens während, vorzugsweise vor der Formung der porösen Vliesstruktur des Implantats, versetzt. Bevorzugt wird das Implantat aus dem Kunststoff, der mit den Partikeln aus dem mindestens einen antimikrobiellen Wirkstoff versehen ist, in einem Arbeitsgang durch Materialauftrag auf einen Träger durch Sprühtechnik ausgebildet. Wie bereits erwähnt, eignet sich als Sprühtechnik die Sprühvliestechnik. Es können Lösungen des bioverträglichen Kunststoffes, die die Partikel aus dem mindestens einen antimikrobiellen Wirkstoff bereits enthalten, ohne Änderung der Sprühtechnik verarbeitet werden. Die Wirkstoffpartikel, insbesondere in Form von Nanopartikeln, lassen sich homogen in der Lösung verteilen.

Weitere Merkmale und Vorteile der Erfindung ergeben sich aus der nachfolgenden Beschreibung von bevorzugten Beispielen In Verbindung mit den Unteransprüchen. Hierbei können die einzelnen Merkmale jeweils für sich alleine oder zu mehreren in Kombination miteinander verwirklicht sein.

### 1. Herstellung einer Polyurethan-Gefäßprothese

1200 g Polyurethangranulat und 13500 g (9 Liter) Chloroform werden in einen Rührtopf gegeben und mit ca. 260 U/min 72 Stunden lang bis zur vollständigen Auflösung gerührt.

Anschließend wird die Lösung mit 73,5 g Nanosilber mit einer Partikelgröße von ca. 50 bis 100 nm (agglomeriert 5 bis 10 µm) versetzt und durch Rühren (3 h) homogenisiert. Die erhaltene Suspension wird über zwei Sprühpistolen (z.B. Krautzberger Typ A-7) bei 3,5 bar Druckluft versprüht. Die Pistolen stehen in einem Abstand von 350 mm zueinander und sind in einem Winkel von 60° zu einer mit 300 U/min rotierenden Walze angeordnet. Es werden zunächst 150 Zyklen mit einer Entfernung der Pistolen zur Walze von 23,5 cm gesprüht, anschließend 280 Zyklen mit einem Abstand von 11 cm zur Walze und zum Abschluss nochmals 100 Zyklen mit einem Abstand von 23, 5 cm. Es kann auch insbesondere bei den verschiedenen Zyklen, mit Suspensionen mit unterschiedlichem Silbergehalt gearbeitet werden. Der Silbergehalt kann insbesondere an mindestens einer Prothesenoberfläche höher sein als im Inneren der Prothesenwandung. Nach Beendigung des Sprühvorgangs werden Walze und Vlies 10 Sekunde n lang in ein organisches Lösungsmittel getaucht, so dass das Vlies in seiner Gesamtheit vollständig benetzt ist. Nach einer Abtropfzeit von 5 min wird das Vlies rotierend getrocknet. Die erhaltene Gefäßprothese hat einen Innendurchmesser von 6 mm und einen Außendurchmesser von 7 mm. Sie besitzt eine leichte Graufärbung.

Der Silbergehalt einer so hergestellten Gefäßprothese beträgt 6,1 ±0,4 Gew.%. Auf ebensolche Weise erhält man bei einem vorgegebenen Silbergehalt der Sprühlösung folgende Silbergehalte der Prothese:
Silbergehalt der Sprühlösung 0,1 % →1,2 Gew.% Silbergehalt der Prothese
Silbergehalt der Sprühlösung 0,5 % →6,1 Gew.% Silbergehalt der Prothese
Silbergehalt der Sprühlösung 1,0 % →12,3 Gew.% Silbergehalt der Prothese

### Porosität und Porengröße PUR Prothese

- Porosität:: 50 % (freies Volumen)
- Porengröße:: 95 % der Poren haben eine Porengröße zwischen 0,1 und 100 µm. Der größte Teil (über 50 % der Poren) hat eine Größe zwischen 1 und 10 µm. Die häufigste Porengröße beträgt 1 µm.

### Wasserdurchtrittsdruck

- Methode:: Die Testprothesen werden mit Wasser gefüllt und mit ansteigendem Druck beaufschlagt. Sobald Wasser auf der externen Oberfläche beobachtet wird, wird der Druck notiert und der Test ist beendet. Dieser Druck ist der Wasserdurchtrittsdruck. Der Wasserdurchtrittsdruck der PUR-Gefäßprothesen liegt bei 333,3 hPa (250 mm Hg) bis 426,6 hPa (320 mm Hg).

### 2. Prüfung auf antimikrobielle Wirksamkeit

### 1) Prüfmethode

Die Prüfkörper werden mit Zellen des Teststamms inkubiert und die Proliferation von Tochterzellen an die Umgebung über die optische Dichte ermittelt.

### Teststamm

Staphylococcus Epideridis 9142 (∼ 10⁶ CFU/ml)

### 2) Durchführung

Die Gefäßprothesen wurden in 7 x 4 mm große Prüfkörper geschnitten und die Untersuchungen an einer Anzahl von n = 8 pro Prüfkörper durchgeführt.

Der Versuch wurde dabei einmal mit und einmal ohne Vorinkubation in Humanplasma durchgeführt. Bei der Vorinkubation in Humanplasma wurden die Proben für 2 h bei 37°C in diesem inkubiert und anschließend mit Phosphatpufferlösung gewaschen.

Alle Prüfkörper wurden zunächst für 1 Stunde bei 37°C mit den Zellen des Teststamms inkubiert, um eine vollständige Adhäsion der Zellen an der Prüfkörperoberfläche zu erreichen. Mittels eines einmaligen Waschens in Phosphatpufferlösung wurden die nicht auf der Oberfläche adhärierten Keime ausgespült. Danach wurden die Prüfkörper zur Entfaltung der antimikrobiellen Eigenschaften weitere 24 Stunden bei 37°C mit den Zellen des Teststamms inkubiert und anschließend aus dem Inkubationsmedium entfernt.

In der verbliebenen Bakteriensuspension wurden über einen Zeitraum von 48 Stunden die proliferierten Tochterzellen optisch erfasst, wobei die erforderliche Zeit bis zur Überschreitung eines bestimmten Schwellenwertes als Wachstumsverzögerung in Stunden angegeben wurde.

### 3) Material

Polyurethanprothesen ausgestattet mit Silber.

Die Gehalte an zugesetztem Nanosilber, bezogen auf die PUR/Chloroform Ausgangslösung, betrugen 0,1 %, 0,5 % und 1,0 % (Gew.% bezogen auf PUR 1,2 %, 6,1 %, 12,3 %).

### 4) Ergebnisse

Die Wachstumsverzögerung der silberhaltigen Prüfmuster ergibt sich aus der gemessen zeitlichen Differenz vom silberhaltigen Prüfkörper zum Referenzwert des nicht silberhaltigen Prüfkörpers.

### Ohne Vorinkubation

| **Probenbezeichnung (Gew.% Ag)** | **Wachstumsverzögerung (h)** | **Ergebnis** |
|---|---|---|
| PUR Kontrolle | 3,9 | nicht antibakteriell |
| PUR 0,1 % | 14,7 | antimikrobiell |
| PUR 0,5 % | 20,5 | antimikrobiell |
| PUR 1,0 % | 33,2 | antimikrobiell |

Mit Vorinkubation in human Plasma

| **Probenbezeichnung (Gew.% Ag)** | **Wachstumsverzögerung (h)** | **Ergebnis** |
|---|---|---|
| PUR Kontrolle | 3,9 | nicht antibakteriell |
| PUR 0,1 % | 16,6 | antimikrobiell |
| PUR 0,5 % | 27,0 | antimikrobiell |
| PUR 1,0 % | 32,1 | antimikrobiell |

Sowohl ohne als auch mit zusätzlicher Vorinkubation in Humanplasma werden bei den nicht silberhaltigen Polyurethanprothesen schon nach kurzer Zeit proliferierte Tochterzellen des Teststammes detektiert, wohingegen es bei den zusätzlich mit Silber ausgestatteten Prothesen zu deutlichen, zeitlichen Wachstumsverzögerungen kommt.

Die antimikrobielle Wirksamkeit ist dabei abhängig von der Silberkonzentration und nimmt somit in der Reihenfolge 0,1 % < 0,5% < 1,0% zu.

## Patentansprüche

1. Antimikrobielles Implantat mit einer flexiblen porösen Vliesstruktur aus einem bioverträglichen Kunststoff, in dem Partikel aus mindestens einem antimikrobiellen Wirkstoff enthalten sind, **dadurch gekennzeichnet, dass** das Implantat als Sprühvlies ausgebildet ist.

2. Implantat nach Anspruch 1, **dadurch gekennzeichnet, dass** es als Hohlorgan, insbesondere als Gefäßprothese, ausgebildet ist.

3. Implantat nach Anspruch 1, **dadurch gekennzeichnet, dass** es als flächiges Implantat, insbesondere als Patch, ausgebildet ist.

4. Implantat nach einem der vorhergehenden Ansprüche, **dadurch gekennzeichnet, dass** die poröse Struktur eine mikroporöse Struktur ist, insbesondere eine solche mit einer Luftdurchlässigkeit von 5 bis 500 ml/cm²/Minute bei einer Druckdifferenz von 1,2 kPa, vorzugsweise von 10 bis 200 ml/cm²/Minute.

5. Implantat nach einem der vorhergehenden Ansprüche, **dadurch gekennzeichnet, dass** der Kunststoff ein nicht resorbierbarer Kunststoff ist.

6. Implantat nach einem der vorhergehenden Ansprüche, **dadurch gekennzeichnet, dass** der Kunststoff ein thermisch plastifizierbarer Kunststoff, insbesondere ein Thermoplast ist.

7. Implantat nach einem der vorhergehenden Ansprüche, **dadurch gekennzeichnet, dass** der Kunststoff ein in Lösungsmittel lösbarer Kunststoff ist.

8. Implantat nach einem der vorhergehenden Ansprüche, **dadurch gekennzeichnet, dass** der Kunststoff ein hydrophober Kunststoff ist.

9. Implantat nach einem der vorhergehenden Ansprüche, **dadurch gekennzeichnet, dass** es dünnwandig ausgebildet ist, insbesondere eine Wandstärke von 0,1 bis 2 mm, vorzugsweise 0,3 bis 0,6 mm, besitzt.

10. Implantat nach einem der vorhergehenden Ansprüche, **dadurch gekennzeichnet, dass** die Partikel aus dem antimikrobiellen Wirkstoff homogen im Kunststoff von Einzelfasern der Vliesstrukturen verteilt sind.

11. Implantat nach einem der Ansprüche 1 bis 10, **dadurch gekennzeichnet, dass** die Partikel aus dem antimikrobiellen Wirkstoff an mindestens einer Oberfläche des Implantats angereichert sind.

12. Implantat nach Anspruch 11, **dadurch gekennzeichnet, dass** an der Oberfläche liegende Fasern der Vliesstruktur eine höhere Konzentration an Partikeln aus dem antimikrobiellen Wirkstoff aufweisen als solche Fasern, die unter der Oberfläche liegen.

13. Implantat nach einem der vorhergehenden Ansprüche, **dadurch gekennzeichnet, dass** die Partikel aus dem mindestens einen antimikrobiellen Wirkstoff eine Größe von 50 bis 100 nm besitzen.

14. Implantat nach einem der vorhergehenden Ansprüche, **dadurch gekennzeichnet, dass** die Partikel aus dem mindestens einen antimikrobiellen Wirkstoff aus mindestens einem antimikrobiell wirkenden Metall, insbesondere aus Silber, bestehen.

15. Implantat nach einem der vorhergehenden Ansprüche, **dadurch gekennzeichnet, dass** der Gehalt an den Partikeln aus dem mindestens einen antimikrobiellen Wirkstoff 0,1 bis 10 Gew.%, insbesondere 0,2 bis 5 Gew.%, beträgt.

16. Verfahren zur Herstellung des Implantates nach einem der vorhergehenden Ansprüche, **dadurch gekennzeichnet, dass** der bioverträgliche Kunststoff mit den Partikeln aus dem mindestens einen antimikrobiellen Wirkstoff spätestens während, vorzugsweise vor der Formung der porösen Struktur des Implantats versetzt wird.

17. Verfahren nach Anspruch 16, **dadurch gekennzeichnet, dass** das Implantat aus dem Kunststoff, der mit den Partikeln aus dem mindestens einen antimikrobiellen Wirkstoff versehen ist, in einem Arbeitsgang, insbesondere durch Materialauftrag auf einen Träger, ausgebildet wird.

## Claims

1. Antimicrobial implant with a flexible porous non-woven structure composed of a biocompatible synthetic material which comprises particles of at least one antimicrobial agent, **characterized in that** the implant is designed as a sprayed non-woven.

2. Implant according to Claim 1, **characterized in that** it is designed as hollow organ, in particular as vascular prosthesis.

3. Implant according to Claim 1, **characterized in that** it is designed as planar implant, in particular as patch.

4. Implant according to any of the preceding claims, **characterized in that** the porous structure is a microporous structure, in particular one with an air permeability of from 5 to 500 ml/cm²/minute with a pressure difference of 1.2 kPa, preferably from 10 to 200 ml/cm²/minute.

5. Implant according to any of the preceding claims, **characterized in that** the synthetic material is a non-absorbable synthetic material.

6. Implant according to any of the preceding claims, **characterized in that** the synthetic material is a thermally plasticizable synthetic material, in particular a thermoplastic.

7. Implant according to any of the preceding claims, **characterized in that** the synthetic material is a synthetic material which can dissolve in solvents.

8. Implant according to any of the preceding claims, **characterized in that** the synthetic material is a hydrophobic synthetic material.

9. Implant according to any of the preceding claims, **characterized in that** it is designed with thin walls, in particular has a wall thickness of from 0.1 to 2 mm, preferably 0.3 to 0.6 mm.

10. Implant according to any of the preceding claims, **characterized in that** the particles of the antimicrobial agent are homogeneously dispersed in the synthetic material of single fibers of the non-woven structures.

11. Implant according to any of Claims 1 to 10, **characterized in that** the particles of the antimicrobial agent are enriched on at least one surface of the implant.

12. Implant according to Claim 11, **characterized in that** fibers of the non-woven structure located on the surface have a higher concentration of particles of the antimicrobial agent than those fibers located underneath the surface.

13. Implant according to any of the preceding claims, **characterized in that** the particles of the at least one antimicrobial agent have a size of from 50 to 100 nm.

14. Implant according to any of the preceding claims, **characterized in that** the particles of the at least one antimicrobial agent consist of at least one metal with antimicrobial activity, in particular of silver.

15. Implant according to any of the preceding claims, **characterized in that** the content of particles of the at least one antimicrobial agent is from 0.1 to 10% by weight, in particular 0.2 to 5% by weight.

16. Process for producing the implant according to any of the preceding claims, **characterized in that** the biocompatible synthetic material is treated with the particles of the at least one antimicrobial agent at the latest during, and preferably before, the shaping of the porous structure of the implant.

17. Process according to Claim 16, **characterized in that** the implant composed of the synthetic material which is provided with the particles of the at least one antimicrobial agent is formed in one operation, in particular by material application to a substrate.

## Revendications

1. Implant antimicrobien comprenant une structure de non-tissé poreuse souple en un plastique biocompatible, qui contient des particules d'au moins un agent actif antimicrobien, **caractérisé en ce que** l'implant est conçu sous la forme d'un non-tissé lié par pulvérisation.

2. Implant selon la revendication 1, **caractérisé en ce qu'**il est conçu sous la forme d'un organe creux, notamment sous la forme d'une prothèse de vaisseau.

3. Implant selon la revendication 1, **caractérisé en ce qu'**il est conçu sous la forme d'un implant plat, notamment sous la forme d'un patch.

4. Implant selon l'une quelconque des revendications précédentes, **caractérisé en ce que** la structure poreuse est une structure microporeuse, notamment présentant une perméabilité à l'air de 5 à 500 ml/cm²/minute à une différence de pression de 1,2 kPa, de préférence de 10 à 200 ml/cm²/minute.

5. Implant selon l'une quelconque des revendications précédentes, **caractérisé en ce que** le plastique est un plastique non résorbable.

6. Implant selon l'une quelconque des revendications précédentes, **caractérisé en ce que** le plastique est un plastique plastifiable thermiquement, notamment un thermoplastique.

7. Implant selon l'une quelconque des revendications précédentes, **caractérisé en ce que** le plastique est un plastique pouvant être dissous dans un solvant.

8. Implant selon l'une quelconque des revendications précédentes, **caractérisé en ce que** le plastique est un plastique hydrophobe.

9. Implant selon l'une quelconque des revendications précédentes, **caractérisé en ce qu'**il est conçu à parois minces, présente notamment une épaisseur de paroi de 0,1 à 2 mm, de préférence de 0,3 à 0,6 mm.

10. Implant selon l'une quelconque des revendications précédentes, **caractérisé en ce que** les particules de l'agent actif antimicrobien sont dispersées de manière homogène dans le plastique par des fibres individuelles des structures de non-tissé.

11. Implant selon l'une quelconque des revendications 1 à 10, **caractérisé en ce que** les particules de l'agent actif antimicrobien sont accumulées sur au moins une surface de l'implant.

12. Implant selon la revendication 11, **caractérisé en ce que** des fibres de la structure de non-tissé se trouvant à la surface présentent une concentration plus élevée en particules de l'agent actif antimicrobien que des fibres se trouvant sous la surface.

13. Implant selon l'une quelconque des revendications précédentes, **caractérisé en ce que** les particules de l'agent actif antimicrobien ou des agents actifs antimicrobiens présentent une taille de 50 à 100 nm.

14. Implant selon l'une quelconque des revendications précédentes, **caractérisé en ce que** les particules de l'agent actif antimicrobien ou des agents actifs antimicrobiens sont constituées d'au moins un métal à action antimicrobienne, notamment d'argent.

15. Implant selon l'une quelconque des revendications précédentes, **caractérisé en ce que** la teneur en particules de l'agent actif antimicrobien ou des agents actifs antimicrobiens est de 0,1 à 10 % en poids, notamment de 0,2 à 5 % en poids.

16. Procédé de fabrication de l'implant selon l'une quelconque des revendications précédentes, **caractérisé en ce que** le plastique biocompatible est mélangé avec les particules de l'agent actif antimicrobien ou des agents actifs antimicrobien au plus tard pendant, de préférence avant la formation de la structure poreuse de l'implant.

17. Procédé selon la revendication 16, **caractérisé en ce que** l'implant est formé à partir du plastique qui est muni des particules de l'agent actif antimicrobien ou des agents actifs antimicrobiens en une étape de travail, notamment par application de matériau sur un support.
